# EUROPEAN PATENT APPLICATION

(11) **EP 1 340 498 A1**
(43) Date of publication of application: **03.09.2003**
(21) Application number: 02004745.2
(22) Date of filing: 01.03.2002
(51) Int. Cl.: A61K 31/428, A61K 31/423, A61K 31/4709, A61K 31/427, A61K 31/4427, A61P 25/28, A61P 35/00

(54) **Use of epothilones in the treatment of brain diseases associated with proliferative processes**

(71) Applicant: Schering Aktiengesellschaft, 13353 Berlin (DE)
(72) Inventor: Lichtner, Rosemarie, 10823 Berlin (DE); Rotgeri, Andrea, 13507 Berlin (DE); Buchmann, Bernd, 16540 Hohen Neuendorf (DE); Hoffmann, Karin, 16567 Mühlenbeck (DE); Klar, Ulrich, 13503 Berlin (DE); Schwede, Wolfgang, 13467 Berlin (DE); Skuballa, Werner, 13465 Berlin (DE)
(74) Representative: Molnia, David

(57) **Abstract**

This invention provides the use of an Epothilone, which shows an average distribution coefficient between plasma and brain of 0.3 to 1.5 in the mouse intravenous bolus injection assay, for the preparation of a medicament for the treatment of a brain disease associated with proliferative processes.

## Description

### Field of the Invention

The present invention relates to the use of Epothilones in the treatment of brain diseases associated with proliferative processes, especially primary or secondary brain tumors, multiple sclerosis, and Alzheimer's disease.

### Background of the Invention

The possibilities of medicamentous treatment of brain diseases are strongly limited by the existence of the so-called blood-brain-barrier (BBB). While the BBB serves as a protective mechanism for preventing exogenous substances to enter the brain tissue, unfortunately, it also prevents the entry of drugs administered by a conventional mode (orally, parenterally, etc.) (A. Maelicke, *Nachr. Chem Tech. Lab.* **1989**, *37,* 32-34).

An important class of brain diseases which are difficult to treat with medicaments for the above-cited reason are diseases associated with proliferative processes such as brain tumors, multiple sclerosis, or Alzheimer's disease. Various studies regarding these diseases, especially cancer, have provided some insights into the efficiency of drug targeting to the brain (W. Shapiro, J. Shapiro, *Semin. Oncol.* **1986**, *13,* 56-69; M. Donelli et al., *Cancer Chemother. Pharmacol.* **1992**, *30,* 251-260). As a rule of thumb, a drug reaches higher concentrations in the brain the lower its molecular mass and the higher its lipophilicity is (C. Unger et al., *Klin. Wochenschr.* **1985**, *63,* 565-571). Nevertheless, it has been found in recent years, that for at least some compounds (M. Fromm, *Int. J. Clin. Pharmacol. Ther.* **2000**, *38,* 69-74) active exclusion mechanisms exist within the BBB, so that drug uptake by brain tissue cannot be simply calculated from physical or chemical data but has to be determined experimentally.

Some experimental methods have been developed to overcome the restrictions of drug uptake by brain tissue caused by the BBB; e.g., direct intrathecal drug application, use of lipid-soluble carriers, or disruption of the BBB by application of high doses of mannitol or other compounds (E. Galanis et al., *Curr. Opin. Neurol.* **2000**, *13,* 619-625; H. Lahrmann et al., J. *Neurol. Neurochir. Psychiatr.* **2001**, *2*, 16-20). These methods are, however, associated with considerable disadvantages and/or undesirable side effects. Most of them can be considered to be in an experimental stage, i.e., they cannot be considered as standard therapies.

As a result of the previous work it can be stated that most cytostatic agents (which is the most important class of drugs for the treatment of diseases associated with proliferative processes) do not reach the same concentration in brain liquor as in blood plasma when applied systemically. For example, it has lately been found that maximum liquor concentrations of 20-30% of the plasma concentrations may be reached when using nitrosoureas, which are considered to be the best BBB penetrating type of cytostatic agents *(Therapiekonzepte Onkologie;* Seeberger, S, Schütte, J. (Eds.), 3^{rd} edition, Springer, Berlin **1998**). Nitrosoureas and a combination of nitrosoureas with procarbazine and vincristine (PCV therapy) are considered to be standard chemotherapeutic agents for the treatment of brain cancer (H. Lahrmann et al., *J. Neurol. Neurochir. Psychiatr.* **2001**, *2*, 16-20; E. Galanis et al., *Curr. Opin. Neurol.* **2000**, *13*, 619-625).

Cytostatic agents can be distinguished according to the mechanism of their pharmacological activity. The most important classes of cytostatic compounds are antimetabolites (e.g. fluorouracil, cytarabine, mercaptopurine), antimitotic agents (e.g. colchicine, paclitaxel, podophyllotoxine, *Vinca*-alkaloids), alkylating agents (e.g. cisplatine, nitrosoureas, nitrogen mustards), antibiotics (e.g. bleomycin), and agents in respect of which the mechanism of their therapeutic effectiveness is not known (e.g. asparaginase).

Although alkylating agents have been found to be useful for cancer treatment, it is an enormous disadvantage of these compounds that their pharmacological mechanism bears a strong carcinogenic potential itself.

In particular nitroso compounds (nitrosoureas and nitroso amines), which were discussed above to be efficient drugs for the treatment of the brain, show these effects: 57 of 60 nitrosoureas (95 %) tested on carcinogenic activity were active (CD Römpp Chemie Lexikon - Version 1.0, Stuttgart/New York: Georg Thieme Verlag **1995**). It would thus be desirable to provide compounds for the efficient treatment of brain diseases associated with proliferative processes which have similar or better BBB-penetrating properties as nitrosoureas, but without their carcinogenic potential.

Within the group of antimitotic agents, Paclitaxel (Taxol®) is the best-known member and one of the best-selling anticancer medicaments in the present time. Unfortunately, paclitaxel has only low ability to penetrate the BBB (M. Glantz et al., *J. Natl. Cancer Inst.* **1995,** *87,* 1077-1081) and is thus not considered to be useful for the treatment of brain diseases via conventional administration routes. Other antimitotic agents, which block the mitotic spindle of a proliferating cell by binding to the spindle-peptide tubulin, and thus cause apoptosis, have been found to be powerful anticancer agents (K.-H. Altmann, *Curr. Opin. Chem. Biol.* **2001**, *5*, 424-431), in respect of which less carcinogenic side effects have been reported than in the case of the alkylating agents discussed above. Epothilones also belong to this group of drugs.

The natural products Epothilone A and B as well as some of their synthetic derivatives have recently found interest in connection with the treatment of cancer, and a lot of work has been done on their synthesis (K. Nicolaou et al., *Angew. Chem.* **1998**, *110,* 2120-2153) and the synthesis of modified structures.

WO 99/07692, WO 99/02514 and WO 99/67252 disclose Epothilone derivatives, their synthesis and pharmaceutical use.

WO 00/66589 deals with the synthesis and pharmaceutical use of Epothilone derivatives having an alkenyl-, alkynyl-, or an cyclic ether containing substituent at the 6-position of the macrocyclic ring.

WO 00/49021 discloses Epothilone derivatives with a halogen substituent in 16-position and their synthesis.

WO 00/71521 discloses a method for the synthesis of olefinic Epothilones.

WO 98/25929 deals with the manufacture of libraries of Epothilone analogs.

WO 99/43320 mentions, in a very general manner, the use of Epothilones for the treatment of cancer. The disclosure focuses on the development of application conditions for the particular compound Epothilone B for the treatment of a wide range of cancer varieties. There is no mention in this document of the difficulties of treating brain diseases associated with proliferative processes as discussed above, or of any specific advantages of using Epothilones in this regard.

It has now unexpectedly been found that certain Epothilones show a particularly good ability to penetrate the BBB compared to other cytostatic agents (antimitotic agents and others), and thus, are particularly useful for the manufacture of medicaments for the treatment of brain diseases associated with proliferative processes. Due to their pharmacological mechanism of action, these compounds can also be used for the treatment of diseases other than cancer, which are associated with proliferative activity.

### Summary of the Invention

Accordingly, the present invention relates to the use of Epothilones for the treatment of brain diseases associated with proliferative processes, or for the preparation of a medicament for the treatment of brain diseases associated with proliferative processes. It also relates to methods of treating brain diseases associated with proliferative processes by oral, rectal, local, or parenteral, preferably inhalational, intravenous, or intraperitoneal, most preferably intravenous administration of an Epothilone.

For the purposes of the present invention, an Epothilone is defined as a cyclic molecule with a 16-membered ring and variable substituents and pharmaceutical activity as a cytostatic agent that binds to tubulin (Asnes et al., *Anal. Biochem.* **1979**, *98,* 64-73; Job et al., *Cellular Pharmacol.* **1993**, I (Suppl. I), S7-S10; Lichtner et al., *PNAS* **2001**, *98,* 11743-11748). The preferred Epothilones for use according to the present invention furthermore show an average distribution coefficient between plasma and brain of 0.3 to 1.5 as measured by the mouse bolus injection assay, as described herein.
A further preferred subgroup is that wherein the Epothilone molecule is a lactone or a lactame molecule.
A preferred subgroup is that wherein the Epothilone shows an average distribution coefficient between plasma and brain of 0.6 to 1.2 in the mouse intravenous bolus injection assay.
A preferred subgroup is the use for the treatment of a brain disease selected from the group consisting of primary brain tumor, secondary brain tumor, Alzheimer's disease and multiple sclerosis.

Preferred Epothilones for use in the present invention are compounds of the general formula: 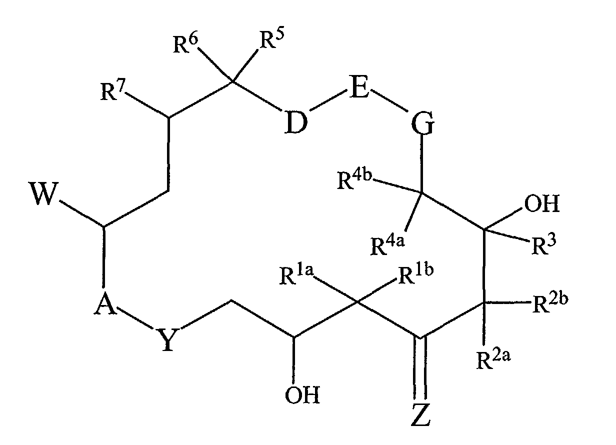 wherein:
- R^{1a}, R^{1b}: are each independently hydrogen, C₁-C₁₀ alkyl, aryl, aralkyl, or together form a -(CH₂)ₘ-group where m is 2 to 5;
- R^{2a}, R^{2b}: are each independently hydrogen, C₁-C₁₀ alkyl, aryl, aralkyl, or together form a -(CH₂)ₙ-group where n is 2 to 5, or C₂-C₁₀ alkenyl, or C₂-C₁₀ alkynyl;
- R³: is hydrogen, C₁-C₁₀ alkyl, aryl, aralkyl;
- R^{4a}, R^{4b}: are each independently hydrogen, C₁-C₁₀ alkyl, aryl, aralkyl, or together form a ―(CH₂)ₚ- group where p is 2 to 5;
- R⁵: is hydrogen, C₁-C₁₀ alkyl, aryl, aralkyl, CO₂H, CO₂alkyl, CH₂OH, CH₂Oalkyl, CH₂Oacyl, CN, CH₂NH₂, CH₂N(alkyl, acyl)_{1,2}, or CH₂Hal;
- R⁶, R⁷: are each hydrogen, or together form an additional bond, or together form an epoxy function;
- G: is O or CH₂;
- D-E: is a group H₂C-CH₂, HC=CH, C=C, CH(OH)-CH(OH), CH(OH)-CH₂, 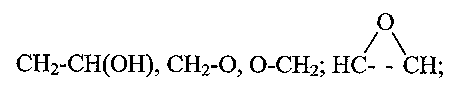
- W: is a group C(=X)R⁸, or is a bi- or tricyclic aromatic or heteroaromatic radical;
- X: is O, or two groups OR²⁰, or a C₂-C₁₀ alkylenedioxy group (which may be straight or branched), or H/OR⁹, or a group CR¹⁰R¹¹;
- R⁸: is hydrogen, C₁-C₁₀ alkyl, aryl, aralkyl, halogen, CN;
- R⁹: is hydrogen or a protecting group PG^{x};
- R¹⁰, R¹¹: are each independently hydrogen, C₁-C₂₀ alkyl, aryl, aralkyl, or together with the methylene carbon form a 5- to 7-membered carbocyclic ring;
- Z: is O or H/OR¹²;
- R¹²: is hydrogen or a protecting group PG^{Z};
- A-Y: is a group O-C(=O), O-CH₂, CH₂-C(=O), NR²¹-C(=O), NR²¹-SO₂;
- R²⁰: is a C₁-C₂₀ alkyl group;
- R²¹: is hydrogen, or C₁-C₁₀ alkyl;
- PG^{X}, PG^{Z}: is C₁-C₂₀ alkyl, C₄-C₇ cycloalkyl, which may contain an oxygen atom in the ring, aryl, aralkyl, C₁-C₂₀ acyl, aroyl, C₁-C₂₀ alkylsulfonyl, arylsulfonyl, tri(C₁-C₂₀ alkyl)silyl, di(C₁-C₂₀ alkyl) arylsilyl, (C₁-C₂₀ alkyl)diarylsilyl, or tri(aralkyl)silyl;
as a single stereoisomer or a mixture of different stereoisomers, and / or as a pharmaceutically acceptable salt thereof.

These compounds are advantageously used in the treatment of, or for the manufacture of a medicament for the treatment of, a brain disease associated with proliferative processes.

In a further embodiment, the present invention relates to a method of treating a brain disease associated with proliferative processes comprising administering to an individual in need thereof a therapeutically effective amount of an Epothilone as defined above.

### Preferred Embodiments

The term "brain disease associated with proliferative processes" as referred to in the context of the present invention includes, but is not limited to, primary brain tumors such as astrocytomas, oligodendrogliomas, pinealomas, medulloblastomas, neurilemmomas, meningeomas, and ependymomas, secondary brain tumors, multiple sclerosis, and Alzheimer's disease, all of which represent preferred brain diseases associated with proliferative processes to be treated in accordance with the present invention.

Particularly preferred brain diseases associated with proliferative processes to be treated by Epothilone administration in accordance with the present invention are primary and secondary brain tumors.

The term "therapeutically effective amount" as used herein refers to that amount of a compound of the invention which, when administered to an individual in need thereof, is sufficient to effect treatment, as defined below, for brain diseases associated with proliferative processes. The amount which constitutes a "therapeutically effective amount" will vary depending on the compound, the disease and its severity, and the age of the human to be treated, but can be determined routinely by one of ordinary skill in the art having regard to his own knowledge and to this disclosure.

"Treating" or "treatment" as used herein refers to the treatment of a brain disease in an individual, which disease is associated with proliferative processes; and include:
(i) preventing the disease from recurring in an individual, in particular, when such individual is in need of further medicamentous treatment after a previous surgical or medicamentous therapy;
(ii) inhibiting the disease, i.e., arresting its development; or
(iii) relieving the disease, i.e., causing regression of the disease.

The term "alkyl" as used herein refers to straight or branched alkyl groups, e. g., methyl, ethyl, propyl, isopropyl, *n*-butyl, *t*-butyl, *n*-pentyl, neopentyl, heptyl, or decyl. Alkyl groups can be perfluorated or substituted by one to five substituents selected from the group consisting of halogen, hydroxy, C₁-C₄ alkoxy, or C₆-C₁₂ aryl (which can be substituted by one to three halogen atoms).

The term "aryl" as used herein refers to an aromatic carbocyclic or heterocyclic moiety containing five to 14 ring atoms, e.g., phenyl, naphthyl, furyl, thienyl, pyridyl, pyrazolyl, pyrimidinyl, oxazolyl, pyridazinyl, pyrazinyl, chinolyl, or thiazolyl. Aryl groups can be substituted by one or more substituents selected from the group consisting of halogen, hydroxy, alkoxy, -CO₂H, -CO₂Alkyl, -NH₂, -NO₂, -N₃, -CN, C₁-C₂₀ alkyl, C₁-C₂₀ acyl, or C₁-C₂₀ acyloxy. The heteroatoms can be oxidized, if this does not cause a loss of aromatic character, e. g., a pyridine moiety can be oxidized to give a pyridine N-oxide.

The term "aralkyl" as used herein refers to a group which can contain up to 14 atoms in the aryl ring (preferred five to ten) and one to eight carbon atoms in the alkyl chain (preferred one to four), e.g., benzyl, phenylethyl, naphthylmethyl, naphthylethyl, furylmethyl, thienylethyl, or pyridylpropyl. The rings can be substituted by one or more substituents selected from the group consisting of halogen, hydroxy, alkoxy, -CO₂H,-CO₂Alkyl, -NH₂, -NO₂, -N₃, -CN, C₁-C₂₀ alkyl, C₁-C₂₀ acyl, or C₁-C₂₀ acyloxy.

The protecting groups PG can be alkyl- and/or aryl-substituted silyl moieties, C₁-C₂₀ alkyl, C₄-C₇ cycloalkyl, which may contain an oxygen atom in the ring, aryl, aralkyl, C₁-C₂₀ acyl, aroyl, alkyl- or arylsulfonyl. Groups which can be easily be removed from the molecule are preferred, e.g., methoxymethyl, methoxyethyl, ethoxyethyl, tetrahydropyranyl, tetrahydrofuranyl, trimethylsilyl, triethylsilyl, t-butyldimethylsilyl, tribenzylsilyl, triisopropylsilyl, benzyl, *p*-nitrobenzyl, *p*-methoxybenzyl, as well as alkylsulfonyl or arylsulfonyl. Preferred acyl groups are formyl, acetyl, propionyl, pivaloyl, butyryl, or benzoyl, which all can be substituted by one or more amino and/or hydroxy moieties.

A preferred group is compounds of the general formula as given above, wherein A-Y is O-C(=O); D-E is H₂C-CH₂; G is CH₂; Z is O; R^{1a}, R^{1b} are both C₁-C₁₀ alkyl or form together a -(CH₂)ₚ- group where p is 2 to 3; R^{2a}, R^{2b} are each independently hydrogen, C₁-C₁₀ alkyl, C₂-C₁₀ alkenyl, or C₂-C₁₀ alkynyl; R³ is hydrogen; R^{4a}, R^{4b} are each independently hydrogen or C₁-C₁₀ alkyl; R⁵ is C₁-C₁₀ alkyl.

Another preferred group is compounds of the general formula as given above, wherein R^{2a}, R^{2b} are each independently hydrogen, C₂-C₁₀ alkenyl or C₂-C₁₀ alkynyl; R⁶, R⁷ form an epoxy function or together form an additional bond; W is a 2-Methylbenzothiazol-5-yl radical or a 2-Methylbenzoxazol-5-yl radical or a Quinoline-7-yl radical.

Of this group, a preferred subgroup is compounds selected from the following:
(4S,7R,8S,9S,13E/Z,16S(E))-4,8-dihydroxy-16-(2-methyl-benzoxazol-5-yl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(1-methyl-2-(2-methyl-benzoxazol-5-yl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-( 1-methyl-2-(2-methyl-benzothiazol-5-yl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(2-methyl-benzothiazol-5-yl)-1-oxa-9,13-dimethyl-5,5-(1,3-trimethylen)-7-(prop-2-en-1-yl)-cyclohexadec-13-ene-2,6-dione;
(1S/R,3S(E),7S,10R,11R, 12S,16R/S)-7,11-dihydroxy-10-(prop-2-en-1-yl)-3-(1-methyl-2-(2-methyl-benzothiazol-5-yl)-12,16-dimethyl-8,8-(1,3-trimethylen)-4,17-dioxabicyclo[14,1,0]heptadecane-5,9-dione;
(4S,7R,8S,9S,13E/Z,16S(E))-4,8-dihydroxy-16-(2-methyl-benzothiazol-5-yl)-1-oxa-5,5,9,13-tetramethyl-7-(prop-2-in-1-yl)-cyclohexadec-13-ene-2,6-dione;
(1S/R,3S(E),7S,10R,11R,12S,16R/S)-7,11-dihydroxy-10-(prop-2-in-1-yl)-3-(1-methyl-2-(2-methyl-benzothiazol-5-yl)-8,8,12,16-tetramethyl-4,17-dioxabicyc1o[ 14.1.0]heptadecane-5,9-dione;
(4S,7R,8S,9S,13E/Z,16S(E))-4,8-dihydroxy-16-(chinolin-2-yl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(1-methyl-2-(chinolin-2-yl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(2-methyl-benzothiazol-5-yl)-1-aza-5,5,9,13-tetramethyl-7-(prop-2-en-1-yl)-cyclohexadec-13-ene-2,6-dione; and
(1S/R,3S(E),7S,10R,11R,12S,16R/S)-7,11-dihydroxy-10-(prop-2-en-1-yl)-3-(1-methyl-2-(2-methyl-benzothiazol-5-yl)-8,8,12,16-tetramethyl-4-aza-17-oxabicyclo[14.1.0]heptadecane-5,9-dione.

Another preferred group of compounds has the general formula as given above, wherein R^{2a}, R^{2b} are each independently hydrogen, or C₁-C₁₀ alkyl; R⁶, R⁷ form an epoxy function, or form an additional bond; W is a group C(=X)R⁸; X is a group CR¹⁰R¹¹; R⁸ is hydrogen, halogen, C₁-C₁₀ alkyl; R¹⁰, R¹¹ are hydrogen/2-methylthiazol-4-yl or hydrogen/2-pyridyl.

Of this group, a preferred subgroup is compounds selected from the following:
(4S,7R,8S,9S,13E/Z,16S(E))-4,8-dihydroxy-16-(1-methyl-2-(2-methyl-4-thiazolyl)ethenyl)-1-oxa-5,5,9,13-tetramethyl-7-ethyl-cyclohexadec-13-ene-2,6-dione;
(1 S/R,3S(E),7S,10R,11R,12S,16R/S)-7,11-dihydroxy-10-ethyl-3-(1-methyl-2-(2-methyl-4-thiazolyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-methyl-4-thiazolyl)ethenyl)-1-oxa-5,5-(1,3-trimethylen)-9,13-dimethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-methyl-4-thiazolyl)ethenyl)-8,8-( 1,3-trimethylen)-12,16-dimethyl-4,17-dioxabicyclo[14.1.0]heptadecane-5,9-dione;
(4S,7R,8S,9S,13E/Z,16S(E))-4,8-dihydroxy-16-(1-methyl-2-(2-methyl-4-thiazolyl)ethenyl)-1-oxa-5,5,9,13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-methyl-4-thiazolyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,7,9,13-pentamethyl-cyclohexadec-13-ene-2,6-dione;
(1 S/R,3 S(Z),7S,10R,11S,12S,16R/S)-7,11-dihydroxy-3-(1-fluor-2-(2-methyl-4-thiazolyl)ethenyl)-8,8,10,12,16-pentamethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,9,13-tetramethyl-7-ethyl-cyclohexadec-13-ene-2,6-dione;
(1S/R,3 S(Z),7S,10R,11S,12S,16R/S)-7,11-dihydroxy-3-(1-fluor-2-(2-methyl-4-thiazolyl)ethenyl)-8,8,12,16-tetramethyl-10-ethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-7,9,13-trimethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8-(1,3-trimethylen)-10,12,16-trimethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-( 1,3-trimethylen)-9,13-dimethyl-7-ethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8-( 1,3-trimethylen)-12,16-dimethyl-10-ethyl-4,17-dioxabicyclo[14.1.0]heptadecane-5,9-dione;
(4S,7R,8S,9S, 13E/Z, 16S(Z))-4,8-dihydroxy-16-(1-chlor-2-(2-methyl-4-thiazolyl)ethenyl)-1-oxa-5,5,7,9,13-pentamethyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,10,12,16-pentamethyl-4,17-dioxabicyclo[14.1.0]heptadecane-5,9-dione;
(4S,7R,8S,9S,13E/Z,16S(Z))-4,8-dihydroxy-16-(1 chlor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S,10R,11S,12S,16R/S)-7,11-dihydroxy-3-(1chlor-2-(2-methyl-4-thiazolyl)ethenyl)-8,8,12,16-tetramethyl-10-ethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-propyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8, 8,12,16-tetramethyl-10-propyl-4,17-dioxabicyclo[14.1.0]heptadecane-5,9-dione;
(4S,7R,8S,9S, 13E/Z,16S(E))-4,8-dihydroxy-16-(1-methyl-2-(2-pyridyl)ethenyl)-1-oxa-5,5,9, 13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5-(1,3-trimethylen)-9,13-dimethyl-7-ethyl-cyc1ohexadec-13-ene-2,6-dione;
(1S/R,3S(E),7S,10R,11R,12S,16R/S)-7,11-dihydroxy-10-ethyl-3-(1-methyl-2-(2-pyridyl)ethenyl)-8,8-(1,3-trimethylen)-12,16-dimethyl-4,17-dioxabicyclo[14.1.0]heptadecane-5,9-dione;
(4S,7R,8S,9S,13E/Z,16 S(E))-4,8-dihydroxy-16-(1-methyl-2-(2-pyridyl)ethenyl)-1-oxa-5,5-(1,3-trimethylen)-7,9,13-trimethyl-cyclohexadec-13-ene-2,6-dione;
(1 S/R,3 S(E),7S,10R,11R,12S,16R/S)-7,11-dihydroxy-3-(1-methyl-2-(2-pyridyl)ethenyl)-8,8-( 1,3-trimethylen)-10,12,16-trimethyl-4,17-dioxabicyclo[14.1.0]heptadecane-5,9-dione;
(4S,7R,8S,9S, 13E/Z,16S(E))-4,8-dihydroxy-16-(1-methyl-2-(2-pyridyl)ethenyl)-1-oxa-5,5,9,13-tetramethyl-7-propyl-cyclohexadec-13-ene-2,6-dione;
(1 S/R,3 S(E),7S, 10R,11R,12S,16R/S)-7,11-dihydroxy-10-propyl-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-7,9,13-trimethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8-(1,3-trimethylen)-10,12,16-dimethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,12,16-tetramethyl-10-ethyl-4,17-dioxabicyclo[14.1.0]heptadecane-5,9-dione;
(4S,7R,8S,9S, 13E/Z,16S(Z))-4,8-dihydroxy-16-(1-fluor-2-(2-methyl-4-thiazolyl)ethenyl)-1-oxa-5,5,9,13-tetramethyl-7-ethyl-cyclohexadec-13-ene-2,6-dione; and
(1S/R,3 S(Z),7S,10R,11S,12S,16R/S)-7,11-dihydroxy-3-(1-fluor-2-(2-methyl-4-thiazolyl)ethenyl)-8,8,12,16-tetramethyl-10-ethyl-4,17-dioxabicyclo[14.1.0]heptadecane-5,9-dione.

Another preferred group is compounds of the general formula as given above, wherein R^{2a}, R^{2b} are each independently hydrogen, C₂-C₁₀ alkenyl or C₂-C₁₀ alkynyl; R⁶, R⁷ form an epoxy function or together form an additional bond; W is a group C(=X)R⁸; X is a group CR¹⁰R¹¹; R⁸ is hydrogen, halogen, C₁-C₁₀ alkyl; R¹⁰, R¹¹ are hydrogen/2-methylthiazol-4-yl or hydrogen/2-pyridyl.

Of this group, a preferred subgroup is compounds selected from the following:
(4S,7R,8S,9S, 13E/Z, 16S(E))-4,8-dihydroxy-16-(1-methyl-2-(2-pyridyl)ethenyl)-1-oxa-5,5,9,13-tetramethyl-7-(prop-2-in-1-yl)-cyclohexadec-13-ene-2,6-dione;
(1S/R,3S(E),7S,10R,11R,12S,16R/S)-7,11-dihydroxy-10-(prop-2-in-1-yl)-3-(2-(2-pyridyl)ethenyl)-8,8,12,16-tetramethyl-4,17-dioxabicyclo[14.1.0]heptadecane-5,9-dione;
(4S,7R, 8S,9S,13 E/Z,16S(E))-4,8-dihydroxy-16-(1-methyl-2-(2-pyridyl)ethenyl)-1-oxa-5, 5,9,13-tetramethyl-7-(prop-2-en-1-yl)-cyclohexadec-13-ene-2, 6-dione;
(1S/R,3 S(E),7S, 10R, 11R,12S,16R/S)-7,11-dihydroxy-10-(prop-2-en-1-yl)-3-(1-methyl-2-(2-pyridyl)ethenyl)-8,8,12,16-tetramethyl-4,17-dioxabicyclo[14.1.0]heptadecane-5,9-dione;
(4S,7R,8S,9S, 13E/Z,16S(E))-4,8-dihydroxy-16-(1-methyl-2-(2-pyridyl)ethenyl)-1-oxa-5,5,9,13-tetramethyl-7-(but-3-in-1-yl)-cyclohexadec-13-ene-2,6-dione;
(1S/R,3S(E),7S,10R,11R,12S,16R/S)-7,11-dihydroxy-10-(but-3-in-1-yl)-3-(1-methyl-2-(2-pyridyl)ethenyl)-8,8,12,16-tetramethyl-4,17-dioxabicyclo[14.1.0]heptadecane-5,9-dione;
(1S/R,3S(E),7S,10R,11R,12S,16R/S)-7,11-dihydroxy-10-(but-3-en-1-yl)-3-(1-methyl-2-(2-pyridyl)ethenyl)-8,8,12,16-tetramethyl-4,17-dioxabicyclo[14.1.0]heptadecane-5,9-dione;
(1S/R,3S(E),7S,10R,11R, 12S,1 6R/S)-7, 11-dihydroxy-10-(but-3-en-1-yl)-3-(2-(2-pyridyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methylthiazol-4-yl)ethenyl)-1-oxa-5,5,9,13-tetramethyl-7-(prop-2-in-1-yl)-cyclohexadec-13-ene-2,6-dione;
(1S/R,3S(Z),7S,10R,11R,12S,16R/S)-7,11-dihydroxy-10-(prop-2-in-1-yl)-3-(1-fluor-2-(2-methylthiazol-4-yl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methylthiazol-4-yl)ethenyl)-1-oxa-5,5,9,13-tetramethyl-7-(prop-2-en-1-yl)-cyclohexadec-13-ene-2,6-dione; and
(1S/R,3S(Z),7S,10R,11R,12S,16R/S)-7,11-dihydroxy-10-(prop-2-en-1-yl)-3-(1-fluor-2-(2-methylthiazol-4-yl)ethenyl)-8,8,12,16-tetramethyl-4,17-dioxabicyclo[14.1.0]heptadecane-5,9-dione.

The synthesis of the compounds listed above is described in the international patent applications WO 99/07692, WO 00/49021, and WO 00/66589, which are incorporated herein by reference.

For the use according to the invention, the compounds can be formulated by methods known in the art. Compositions for the oral, rectal, parenteral or local application can be prepared in the form of tablets, capsules, granulates, suppositories, implantates, sterile injectable aqueous or oily solutions, suspensions or emulsions, aerosols, salves, creams, or gels, retard preparations or retard implantates. The compounds may also be administered by implantable dosing systems.

The pharmaceutical active compound(s) can thus be mixed with adjuvants known in the art, such as gum arabic, talcum, starch, mannitol, methyl cellulose, lactose, surfactants such as tweens® or myrj®, magnesium stearate, aqueous or non-aqueous carriers, paraffin derivatives, wetting agents, dispersing agents, emulsifiers, preservatives, and flavors.

The compounds can be used in the form of their clathrates of α-, β-, or γ-cyclodextrin or of substituted α-, β-, or γ-cyclodextrines, or in the form of a liposomal composition, in particular a liposomal composition comprising a polyethyleneglycol(PEG)-derivatized lipid.

The invention also relates to pharmaceutical compositions containing one or more of the pharmaceutically active compounds listed above, and their use for the treatment and in the methods in accordance with the present invention. Preferably, one dose unit of these compositions contains about 0.01-100 mg of the pharmaceutically active compound(s). The dosage for the use according to the invention for a human is about 0.01-100 mg per day; a preferred dosage is about 0.02-70 mg per day; a more preferred dosage is about 0.04-40 mg per day.

### Brief Description of the Figures

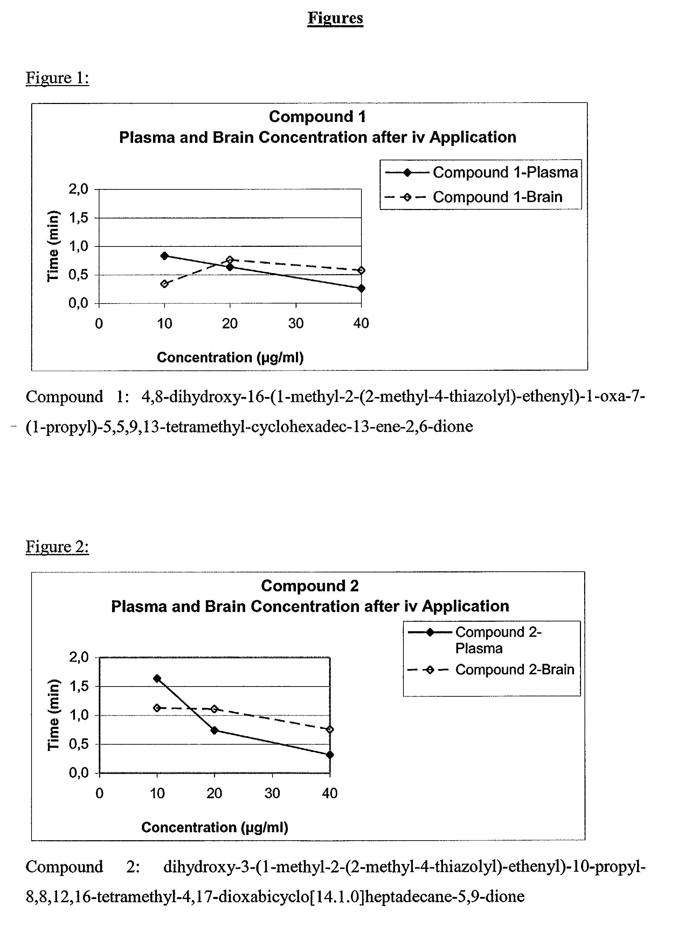

Figure 1 shows the plasma and brain concentrations of4,8-dihydroxy-16-(1-methyl-2-(2-methyl-4-thiazolyl)-ethenyl)-1-oxa-7-(1-propyl)-5,5,9,13-tetranethyl-cyclohexadec-13-ene-2,6-dione (compound 1) after iv application, monitored over a period of 40 min, determined in the animal model of the Example.
Figure 2 shows the plasma and brain concentrations of ³H-labeled dihydroxy-3-(1-methyl-2-(2-methyl-4-thiazolyl)-ethenyl)-10-propyl-8,8,12,16-tetramethyl-4,17-dioxabicyclo[14.1.0]heptadecane-5,9-dione (compound 2) after iv application, monitored over a period of 40 min, determined in the animal model of the Example.

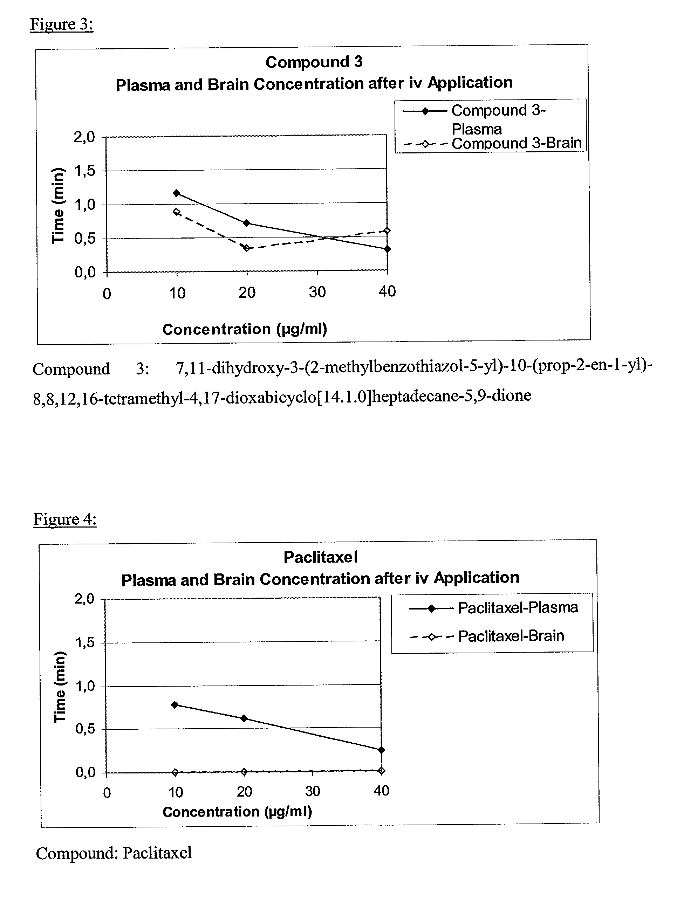

Figure 3 shows the plasma and brain concentrations of ³H-labeled 7,11-dihydroxy-3-(2-methylbenzothiazol-5-yl)-10-(prop-2-en-1-yl)-8,8,12,16-tetramethyl-4,17-dioxabicyclo[14.1.0]heptadecane-5,9-dione (compound 3) after iv application, monitored over a period of 40 min, determined in the animal model of the Example.
Figure 4 shows the plasma and brain concentrations of ³H-labeled paclitaxel after iv application, monitored over a period of 40 min, determined in the animal model of the Example.

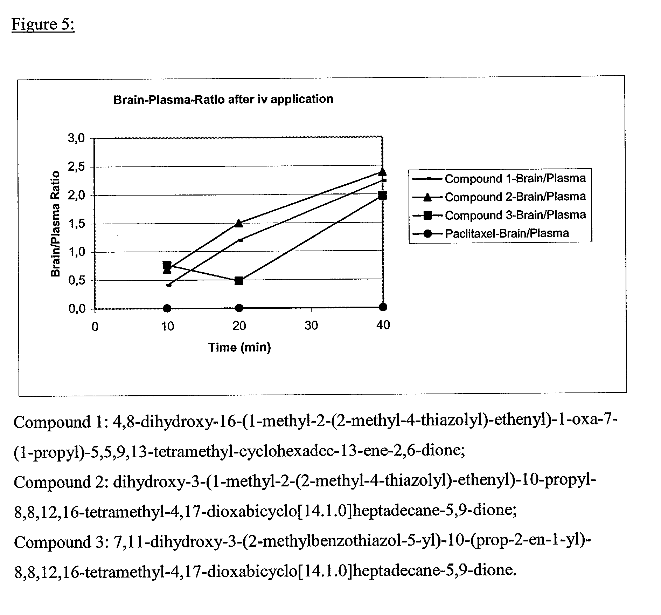

Figure 5 shows the brain-plasma-ratio after iv application of the Epothilones of figures 1-3 and paclitaxel as comparison, monitored over a period of 40 minutes, derived from the data of figures 1-4.

### Example (Mouse bolus injection assay)

### (In vivo assay for the evaluation of blood and brain levels of Epothilones)

Male SCID mice (20-25 g, non-leaky) were treated with a single dose of tritium-labeled Epothilones and paclitaxel (5 mg/kg; 7.4 MBq/mg; in 30 % Hydroxypropyl-β-cyclodextrin (HPβCD)/NaC1 iv bolus injection). Partitioning of radioactivity between blood and brain was measured by liquid scintillation counting (LSC) and HPLC-radioflow at three time points (10, 20 and 40 min) after injection.

The following compounds were tested in this assay:
Paclitaxel;
compound 1: 4,8-dihydroxy-16-(1-methyl-2-(2-methyl-4-thiazolyl)-ethenyl)-1-oxa-7-(1-propyl)-5,5,9,13-tetramethyl-cyclohexadec-13-ene-2,6-dione;
compound 2: dihydroxy-3-(1-methyl-2-(2-methyl-4-thiazolyl)-ethenyl)-10-propyl-8,8,12,16-tetramethyl-4,17-dioxabicyclo[14.1.0]heptadecane-5,9-dione; and
compound 3: 7,11-dihydroxy-3-(2-methylbenzothiazol-5-yl)-10-(prop-2-en-1-yl)-8,8,12,16-tetramethyl-4,17-dioxabicyclo[14.1.0]heptadecane-5,9-dione.

### Results:

All Epothilones were found in the brain at 40 min after iv application in concentrations that exceeded the plasma concentration. For compound 1 and 2 a higher brain plasma ratio was already observed after 20 min. For compound 3 at 10 and 20 minutes a high variation between the animals within one group was observed. 40 minutes after application paclitaxel was detected in the brain in considerable amounts, too.

When comparing the partial (0-40 min) areas under the plasma/brain level time curve, a ratio AUCbrain/AUCplasma of approx. 1 was found (compound 1: 1.0; compound 2: 1.2; compound 3: 0.8) indicating a free access to the brain.

Paclitaxel was below the limit of quantitation in all brain samples but in comparable concentrations in plasma leading to a AUCbrain/AUCplasma ratio of zero.

Concentrations measured for these compounds and AUC ratios calculated thereof are summarized in table 1.

### Conclusion:

In contrast to paclitaxel, Epothilones seem to penetrate the blood-brain-barrier to a significant extend. Persistance in the brain is longer compared to plasma.

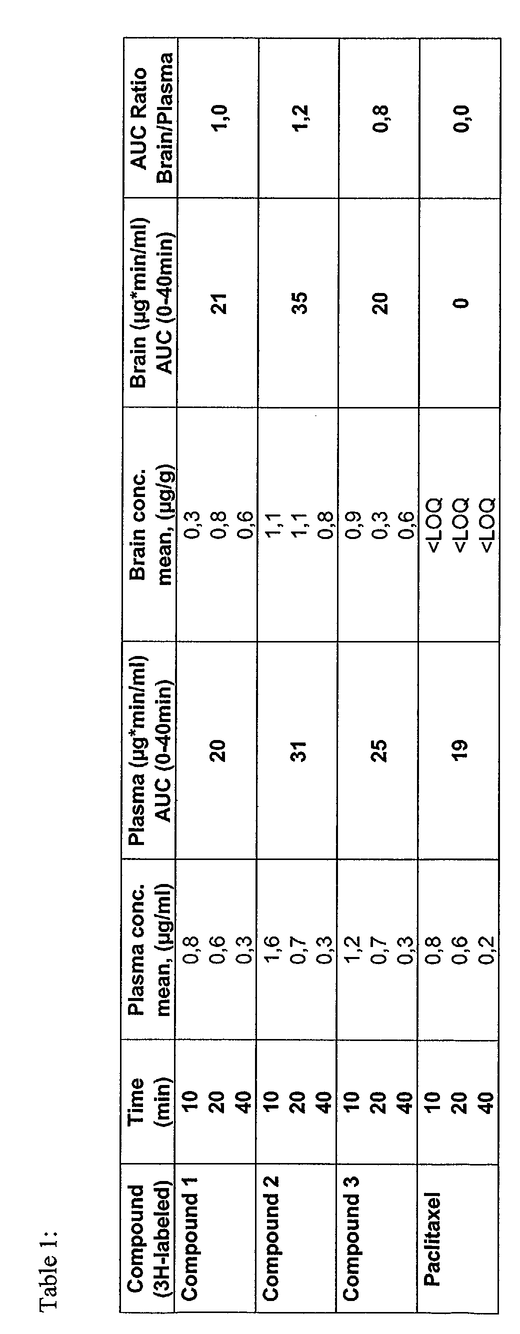

## Claims

1. Use of an Epothilone, which shows an average distribution coefficient between plasma and brain of 0.3 to 1.5 in the mouse intravenous bolus injection assay, for the preparation of a medicament for the treatment of a brain disease associated with proliferative processes.

2. The use of claim 1, wherein the Epothilone is a lactone or a lactame molecule.

3. The use of claim 2, wherein the average distribution coefficient between plasma and brain is 0.6 to 1.2.

4. Use of a compound of the general formula: 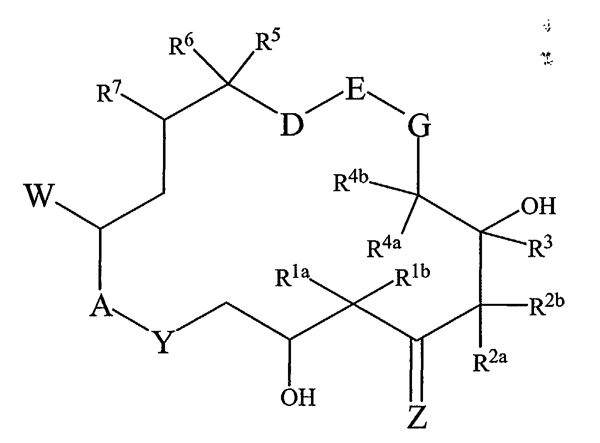 wherein
R^{1a} R^{1b} are each independently hydrogen, C₁-C₁₀ alkyl, aryl, aralkyl, or together form a -(CH₂)ₘ-group where m is 2 to 5;
R^{2a}, R^{2b} are each independently hydrogen, C₁-C₁₀ alkyl, aryl, aralkyl, or together form a -(CH₂)ₙ-group where n is 2 to 5, or C₂-C₁₀ alkenyl, or C₂-C₁₀ alkynyl;
R³ is hydrogen, C₁-C₁₀ alkyl, aryl, aralkyl;
R^{4a}, R^{4b} are each independently hydrogen, C₁-C₁₀ alkyl, aryl, aralkyl, or together form a -(CH₂)ₚ- group where p is 2 to 5;
R⁵ is hydrogen, C₁-C₁₀ alkyl, aryl, aralkyl, CO₂H, CO₂alkyl, CH₂OH, CH₂Oalkyl, CH₂Oacyl, CN, CH₂NH₂, CH₂N(alkyl, acyl)_{1,2}, or CH₂Hal;
R⁶, R⁷ are each hydrogen, or together form an additional bond, or together form an epoxy function;
G is O or CH₂;
D-E is a group H₂C-CH₂, HC=CH, C≡C, CH(OH)-CH(OH), CH(OH)-CH₂, 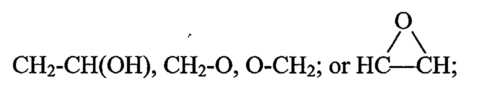
W is a group C(=X)R⁸, or is a bi- or tricyclic aromatic or heteroaromatic radical;
X is O, or two groups OR²⁰, or a C₂-C₁₀ alkylenedioxy group (which may be straight or branched), or H/OR⁹, or a group CR¹⁰R¹¹;
R⁸ is hydrogen, C₁-C₁₀ alkyl, aryl, aralkyl, halogen, CN;
R⁹ is hydrogen or a protecting group PG^{X};
R¹⁰, R¹¹ are each independently hydrogen, C₁-C₂₀ alkyl, aryl, aralkyl, or together with the methylene carbon form a 5- to 7-membered carbocyclic ring;
Z is O or H/OR¹²;
R¹² is hydrogen or a protecting group PG^{Z};
A-Y is a group O-C(=O), O-CH₂, CH₂-C(=O), NR²¹-C(=O), or NR²¹-SO₂;
R²⁰ is a C₁-C₂₀ alkyl group;
R²¹ is hydrogen, or C₁-C₁₀ alkyl;
PG^{X}, PG^{Z} is C₁-C₂₀ alkyl, C₄-C₇ cycloalkyl, which may contain an oxygen atom in the ring, aryl, aralkyl, C₁-C₂₀ acyl, aroyl, C₁-C₂₀ alkylsulfonyl, arylsulfonyl, tri(C₁-C₂₀ alkyl)silyl, di(C₁-C₂₀ alkyl) arylsilyl, (C₁-C₂₀ alkyl) diarylsilyl, or tri(aralkyl)silyl;
as a single stereoisomer or a mixture of different stereoisomers,
and / or as a pharmaceutically acceptable salt thereof,
for the preparation of a medicament for the treatment of a brain disease associated with proliferative processes.

5. The use of any one of claims 1-4, where the brain disease is selected from the group consisting of primary brain tumor, secondary brain tumor, Alzheimer's disease and multiple sclerosis.

6. The use of any one of claims 4 or 5, wherein
A-Y is O-C(=O);
D-E is H₂C-CH₂;
G is CH₂;
Z is O;
R^{1a}, R^{1b} are both C₁-C₁₀ alkyl or together form a -(CH₂)ₘ- group where m is 2 or 3;
R^{2a}, R^{2b} are each independently hydrogen, C₁-C₁₀ alkyl, C₂-C₁₀ alkenyl, or C₂-C₁₀ alkynyl;
R³ is hydrogen;
R^{4a}, R^{4b} are each independently hydrogen or C₁-C₁₀ alkyl;
R⁵ is C₁-C₁₀ alkyl.

7. The use of any one of claims 4-6, wherein
R^{2a}, R^{2b} are each independently hydrogen, C₂-C₁₀ alkenyl or C₂-C₁₀ alkynyl;
R⁶, R⁷ together form an epoxy function or an additional bond; and
W is a 2-Methylbenzothiazol-5-yl radical, a 2-Methylbenzoxazol-5-yl radical, or a Quinoline-7-yl radical.

8. The use of claim 7, wherein the compound is selected from the group consisting of:
(4S,7R,8S,9S,13E/Z,16S(E))-4,8-dihydroxy-16-(2-methyl-benzoxazol-5-yl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(1-methyl-2-(2-methyl-benzoxazol-5-yl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(2-methyl-benzothiazol-5-yl)-1-oxa-5,5,9,13-tetramethyl-7-(prop-2-en-1-yl)-cyclohexadec-13-ene-2,6-dione;
(1S/R,3S(E),7S, 10R, 11R,12S,16R/S)-7,11-dihydroxy-10-(prop-2-en-1-yl)-3-(1-methyl-2-(2-methyl-benzothiazol-5-yl)-8,8,12,16-tetramethyl-4,17-dioxabicyclo[14.1.0]heptadecane-5,9-dione;
(4S,7R,8S,9S, 13E/Z,16S(E))-4,8-dihydroxy-16-(2-methyl-benzothiazol-5-yl)-1-oxa-9,13-dimethyl-5,5-( 1,3-trimethylen)-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(1-methyl-2-(2-methyl-benzothiazol-5-yl)-12,16-dimethyl-8,8-(1,3-trimethylen)-4,17-dioxabicyclo[14.1.0]heptadecane-5,9-dione;
(4S,7R,8S,9S,13E/Z,16S(E))-4,8-dihydroxy-16-(2-methyl-benzothiazol-5-yl)-1-oxa-5,5,9,13-tetramethyl-7-(prop-2-in-1-yl)-cyclohexadec-13-ene-2,6-dione;
(1S/R,3S(E),7S,10R,11R,12S,16R/S)-7,11-dihydroxy-10-(prop-2-in-1-yl)-3-(1-methyl-2-(2-methyl-benzothiazol-5-yl)-8,8,12,16-tetramethyl-4,17-dioxabicyclo[ 14.1 .0]heptadecane-5,9-dione;
(4S,7R,8S,9S,13E/Z,16S(E))-4,8-dihydroxy-16-(chinolin-2-yl)-1-oxa-5,5,9,13-tetramethyl-7-(prop-2-en-1-yl)-cyclohexadec-13-ene-2,6-dione;
(1S/R,3S(E),7S,10R,11R,12S,16R/S)-7,11-dihydroxy-10-(prop-2-en-1-yl)-3-(1-methyl-2-(chinolin-2-yl)-8,8,12,16-tetramethyl-4,17-dioxabicyclo[ 14.1.0]heptadecane-5,9-dione;
(4S,7R,8S,9S,13E/Z,16S(E))-4,8-dihydroxy-16-(2-methyl-benzothiazol-5-yl)-1-aza-5,5,9,13-tetramethyl-7-(prop-2-en-1-yl)-cyclohexadec-13-ene-2,6-dione; and
(1S/R,3S(E),7S,10R,11R,12S,16R/S)-7,11-dihydroxy-10-(prop-2-en-1-yl)-3-(1-methyl-2-(2-methyl-benzothiazol-5-yl)-8,8,12,16-tetramethyl-4-aza-17-oxabicyclo[ 14.1.0]heptadecane-5,9-dione.

9. The use of any one of claims 4-6, wherein
R^{2a}, R^{2b} are each independently hydrogen, or C₁-C₁₀ alkyl;
R⁶, R⁷ together form an epoxy function or an additional bond;
W is a group C(=X)R⁸;
X is a group CR¹⁰R¹¹;
R⁸ is hydrogen, halogen, or C₁-C₁₀ alkyl; and
R¹⁰, R¹¹ are hydrogen/2-methylthiazol-4-yl or hydrogen/2-pyridyl.

10. The use of claim 9, wherein the compound is selected from the group consisting of:
(4S,7R,8S,9S, 13E/Z,16S(E))-4,8-dihydroxy-16-(1-methyl-2-(2-methyl-4-thiazolyl)ethenyl)-1-oxa-5,5,9,13-tetramethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-methyl-4-thiazolyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-methyl-4-thiazolyl)ethenyl)-1-oxa-5,5-(1,3-trimethylen)-9,13-dimethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-methyl-4-thiazolyl)ethenyl)-8,8-(1,3-trimethylen)-12,16-dimethyl-4,17-dioxabicyc1o[14.1.0]heptadecane-5,9-dione;
(4S,7R,8S,9S,13E/Z,16S(E))-4,8-dihydroxy-16-(1-methyl-2-(2-methyl-4-thiazolyl)ethenyl)-1-oxa-5,5,9,13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-methyl-4-thiazolyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5,7,9,13-pentamethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8,10,12,16-pentamethyl-4,17-dioxabicyclo[14.1.0]heptadecane-5,9-dione;
(4S,7R.8S,9S, 13E/Z, 16S(Z))-4,8-dihydroxy-16-(1-fluor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8,12,16-tetramethyl-10-ethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-7,9,13-trimethyl-cyclohexadec-13-ene-2,6-dione;
(1S/R,3S(Z),7S,10R,11S,12S,16R/S)-7,11-dihydroxy-3-(1-fluor-2-(2-methyl-4-thiazolyl)ethenyl)-8,8-(1,3-trimethylen)-10,12,16-trimethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-9,13-dimethyl-7-ethyl-cyclohexadec-13-ene-2,6-dione;
(1 S/R,3S(Z),7S,10R, 11S,12S,16R/S)-7,11-dihydroxy-3-(1-fluor-2-(2-methyl-4-thiazolyl)ethenyl)-8,8-( 1,3-trimethylen)-12,16-dimethyl-10-ethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,7,9,13-pentamethyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,10,12,16-pentamethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-ethyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,12,16-tetramethyl-10-ethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-propyl-cyclohexadec-13-ene-2,6-dione;
(1S/R,3S(Z),7S,10R,11S,12S,16R/S)-7,11-dihydroxy-3-(1-chlor-2-(2-methyl-4-thiazolyl)ethenyl)-8,8,12,16-tetramethyl-10-propyl-4,17-dioxabicyclo[ 14.1 .0]heptadecane-5,9-dione;
(4S,7R,8S,9S, 13E/Z, 1 6S(E))-4,8-dihydroxy-16-(1-methyl-2-(2-pyridyl)ethenyl)-1-oxa-5,5,9,13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5-(1,3-trimethylen)-9,13-dimethyl-7-ethyl-cyclohexadec-13-ene-2,6-dione;
(1S/R,3S(E),7S,10R,11R,12S,16R/S)-7,11-dihydroxy-10-ethyl-3-(1-methyl-2-(2-pyridyl)ethenyl)-8,8-(1,3-trimethylen)-12,16-dimethyl-4,17-dioxabicyclo[14.1.0]heptadecane-5,9-dione;
(4S,7R,8S,9S, 13E/Z,16S(E))-4,8-dihydroxy-16-(1-methyl-2-(2-pyridyl)ethenyl)-1-oxa-S,S-( 1,3-trimethylen)-7,9,13-trimethyl-cyclohexadec-13-ene-2,6-dione;
(1S/R,3S(E),7S,10R,11R,12S,16R/S)-7,11-dihydroxy-3-(1-methyl-2-(2-pyridyl)ethenyl)-8,8-(1,3-trimethylen)-10,12,16-trimethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-propyl-cyclohexadec-13-ene-2,6-dione;
(1S/R,3S(E),7S,10R,11R,12S,16R/S)-7,11-dihydroxy-10-propyl-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methyl-4-thiazolyl)ethenyl)-1-oxa-5,5-(1,3-trimethylen)-7,9,13-trimethyl-cyclohexadec-13-ene-2,6-dione;
(1 S/R,3S(Z),7S,10R,11S,12S,16R/S)-7,11-dihydroxy-3-(1-fluor-2-(2-methyl-4-thiazolyl)ethenyl)-8,8-(1,3-trimethylen)-10,12,16-dimethyl-4,17-dioxabicyclo[14.1.0]heptadecane-5,9-dione;
(4S,7R,8S,9S,13E/Z,16S(Z))-4,8-dihydroxy-16-(1-chlor-2-(2-methyl-4-thiazolyl)ethenyl)-1-oxa-5,5,9,13-tetramethyl-7-ethyl-cyclohexadec-13-ene-2,6-dione;
(1S/R,3 S(Z),7S,10R,11S,12S,16R/S)-7,11-dihydroxy-3-(1-chlor-2-(2-methyl-4-thiazolyl)ethenyl)-8,8,12,16-tetramethyl-10-ethyl-4,17-dioxabicyclo[14.1.0]heptadecane-5,9-dione;
(4S,7R,8S,9S, 13E/Z, 16S(Z))-4,8-dihydroxy-16-(1-fluor-2-(2-methyl-4-thiazolyl)ethenyl)-1-oxa-5,5,9,13-tetramethyl-7-ethyl-cyclohexadec-13-ene-2,6-dione; and
(1S/R,3 S(Z),7S,10R,11S,12S,16R/S)-7,11-dihydroxy-3-(1-fluor-2-(2-methyl-4-thiazolyl)ethenyl)-8,8,12,16-tetramethyl-10-ethyl-4,17-dioxabicyclo[14.1.0]heptadecane-5,9-dione.

11. The use of any one of claims 4-6, wherein
R^{2a}, R^{2b} are each independently hydrogen, C₂-C₁₀ alkenyl or C₂-C₁₀ alkynyl;
R⁶, R⁷ together form an epoxy function or an additional bond;
W is a group C(=X)R⁸;
X is a group CR¹⁰R¹¹;
R⁸ is hydrogen, halogen, or C₁-C₁₀ alkyl; and
R¹⁰, R¹¹ are hydrogen/2-methylthiazol-4-yl or hydrogen/2-pyridyl.

12. The use of claim 11, wherein the compound is selected from the group consisting of:
(4S,7R,8S,9S, 13E/Z, 16S(E))-4,8-dihydroxy-16-(1-methyl-2-(2-pyridyl)ethenyl)-1-oxa-5,5,9,13-tetramethyl-7-(prop-2-in-1-yl)-cyclohexadec-13-ene-2,6-dione;
(1S/R,3S(E),7S,10R,11R,12S,16R/S)-7,11-dihydroxy-10-(prop-2-in-1-yl)-3-(2-(2-pyridyl)ethenyl)-8,8,12,16-tetramethyl-4,17-dioxabicyclo[ 14.1 .0]heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-(prop-2-en-1-yl)-cyclohexadec-13-ene-2,6-dione;
(1 S/R,3S(E),7S,10R,11R,12S,16R/S)-7,11-dihydroxy-10-(prop-2-en-1-yl)-3-(1-methyl-2-(2-pyridyl)ethenyl)-8,8,12,16-tetramethyl-4,17-dioxabicyclo[14.1.0]heptadecane-5,9-dione;
(4S,7R,8S,9S,13E/Z,16S(E))-4,8-dihydroxy-16-(1-methyl-2-(2-pyridyl)ethenyl)-1-oxa-5,5,9,13-tetramethyl-7-(but-3-in-1-yl)-cyclohexadec-13-ene-2,6-dione;
(1S/R,3S(E),7S,10R,11R,12S,16R/S)-7,11-dihydroxy-10-(but-3-in-1-yl)-3-(1-methyl-2-(2-pyridyl)ethenyl)-8,8,12,16-tetramethyl-4,17-dioxabicyclo[14.1.0]heptadecane-5,9-dione;
(1S/R,3S(E),7S,10R,11R,12S,16R/S)-7,11-dihydroxy-10-(but-3-en-1-yl)-3-(1-methyl-2-(2-pyridyl)ethenyl)-8,8,12,16-tetramethyl-4,17-dioxabicyclo[14.1.0]heptadecane-5,9-dione;
(1 S/R,3S(E),7S,10R,11R,12S,16R/S)-7,11-dihydroxy-10-(but-3-en-1-yl)-3-(2-(2-pyridyl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methylthiazol-4-yl)ethenyl)-1-oxa-5,5,9,13-tetramethyl-7-(prop-2-in-1-yl)-cyclohexadec-13-ene-2,6-dione;
(1S/R,3S(Z),7S,10R,11R,12S,16R/S)-7,11-dihydroxy-10-(prop-2-in-1-yl)-3-(1-fluor-2-(2-methylthiazol-4-yl)ethenyl)-8,8,12,16-tetramethyl-4,17-dioxabicyclo[14.1.0]heptadecane-5,9-dione;
(4S,7R,8S,9S,13E/Z,16S(Z))-4,8-dihydroxy-16-(1-fluor-2-(2-methylthiazol-4-yl)ethenyl)-1-oxa-5,5,9,13-tetramethyl-7-(prop-2-en-1-yl)-cyclohexadec-13-ene-2,6-dione; and
(1S/R,3S(Z),7S,10R,11R,12S,16R/S)-7,11-dihydroxy-10-(prop-2-en-1-yl)-3-(1-fluor-2-(2-methylthiazol-4-yl)ethenyl)-8,8,12,16-tetramethyl-4,17-dioxabicyclo[14.1.0]heptadecane-5,9-dione.

13. A method of treating a brain disease associated with proliferative processes comprising administering to an individual in need thereof a therapeutically effective amount of an Epothilone as defined in any one of claims 1 to 12.

14. The use or the method according to any one of claims 1 to 12, wherein the medicament or the Epothilone is to be administered orally, parenterally, rectally, or locally.
